# EUROPEAN PATENT APPLICATION

(11) **EP 4 272 662 A1**
(43) Date of publication of application: **08.11.2023**
(21) Application number: 23170821.5
(22) Date of filing: 28.04.2023
(51) Int. Cl.: A61B 17/34, A61B 17/08, A61B 17/10, A61B 34/37, A61B 90/50, A61B 17/122, A61B 17/128

(54) **SURGICAL ROBOTIC SYSTEMS AND RELOADING MODULES THEREOF**

(30) Priority: 02.05.2022 US 202263337238 P; 02.03.2023 US 202318116332
(71) Applicant: Covidien LP, Mansfield, MA 02048 (US)
(72) Inventor: FISCHVOGT, Gregory W., Reno, 89521 (US); SNIFFIN, Kevin S., North Haven, 06473 (US); THOMAS, Jonathan D., North Haven, 06473 (US)
(74) Representative: Maschio, Antonio

(57) **Abstract**

A surgical robotic system for use in a minimally invasive surgical procedure includes a trocar for facilitating passage of a surgical instrument into a body cavity, and a reloading module configured to couple to a head of the trocar. The reloading module defines a channel that is coaxial with a channel of the trocar and includes a cartridge storing a plurality of surgical implants. The reloading module allows for an expedited loading of the surgical implants thereof onto an end effector of a surgical instrument.

## Description

### CROSS-REFERENCE TO RELATED APPLICATIONS

The present application claims the benefit of and priority to U.S. Provisional Patent Application Serial No. 63/337,238, filed on May 2, 2022, the entire content of which being hereby incorporated by reference.

### FIELD

The present technology is generally related to surgical robotic systems used in minimally invasive medical procedures.

### BACKGROUND

Some surgical robotic systems include a console supporting a surgical robotic arm and a surgical instrument or at least one end effector (e.g., forceps or a grasping tool) mounted to the robotic arm. The robotic arm provides mechanical power to the surgical instrument for its operation and movement. Each robotic arm may include an instrument drive unit operatively connected to the surgical instrument and coupled to the robotic arm via a rail. In operation, the robotic arm is moved to a position over a patient and then guides the surgical instrument into a small incision via a surgical trocar or a natural orifice of a patient to position the end effector at a work site within the patient's body. The surgical trocar may be attached to an end of the surgical robotic arm and held in a fixed position during insertion of the surgical instrument therethrough.

Depending on the surgical robotic procedure, a spent surgical instrument may need to be reloaded or exchanged with another surgical instrument. Exchanging a surgical instrument during a procedure is often time-consuming by requiring a complex coordinated effort amongst hospital staff including detachment of the surgical instrument from the robotic arm, reattachment of a loaded surgical instrument, and calibration of the surgical instrument.

It would be advantageous to provide an apparatus that expedites reloading of a surgical instrument during a surgical robotic procedure.

### SUMMARY

In one aspect of the disclosure, a surgical robotic system is provided and includes a surgical robotic arm, a trocar, and a reloading module. The surgical robotic arm has an elongated rail configured to movably support a surgical instrument. The trocar includes a head configured for attachment to the elongated rail, and a cannula extending distally from the head and configured to receive the surgical instrument. The reloading module is configured to couple to the head of the trocar and includes a housing and a cartridge movable coupled to the housing. The housing of the reloading module defines a longitudinal channel configured to be coaxial with the cannula of the trocar when the reloading module is coupled to the head of the trocar. The cartridge is configured to store a plurality of surgical implants and is configured to move relative to the housing to position a surgical implant of the plurality of surgical implants within the longitudinal channel of the housing.

In aspects, the cartridge may be rotatably supported in the housing such that the cartridge rotates relative to the housing from a first position to a second position. In the first position, the surgical implant may be positioned outside of the longitudinal channel of the housing. In the second position, the surgical implant mat be within the longitudinal channel of the housing.

In aspects, the cartridge may be annular and may include a plurality of circumferentially spaced flanges that detachably support the respective plurality of surgical implants.

In aspects, the cartridge may define at least one slot configured to be coaxial with the longitudinal channel of the housing when the cartridge is in the first position.

In aspects, the at least one slot may be a plurality of slots. Each slot may be positioned between adjacent flanges of the plurality of flanges.

In aspects, the reloading module may further include a motor operably coupled to the cartridge and configured to move the cartridge to change the position of the surgical implant relative to the longitudinal channel of the housing.

In aspects, the reloading module may further include a sensor in communication with the motor. The motor may be configured to move the cartridge to position the surgical implant within the longitudinal channel when the sensor determines that a distal end portion of the surgical instrument is moved proximally of the cartridge.

In aspects, the surgical implants may include staples, clips, and/or sutures.

In accordance with another aspect of the disclosure, a surgical robotic system is provided that includes a trocar and a reloading module. The trocar includes a head configured for attachment to a surgical robotic arm, and a cannula extending distally from the head and configured to receive a surgical instrument. The reloading module is configured to couple to the head of the trocar and includes a housing and a cartridge rotatably coupled to the housing. The housing defines a longitudinal channel configured to be coaxial with the cannula of the trocar when the reloading module is coupled to the head of the trocar. The cartridge is configured to store a plurality of surgical implants and is configured to rotate relative to the housing between a first position and a second position. In the first position, a surgical implant of the plurality of surgical implants is outside of the longitudinal channel of the housing. In the second position, the surgical implant is within the longitudinal channel of the housing.

In aspects, the reloading module may further include a motor operably coupled to the cartridge and configured to rotate the cartridge between the first and second positions.

In aspects, the reloading module may further include a sensor in communication with the motor. The motor may be configured to rotate the cartridge from the first position to the second position when the sensor determines that a distal end portion of the surgical instrument is moved proximally of the cartridge.

In accordance with another aspect of the disclosure, a reloading module for use in a surgical robotic system is provided that includes a housing, a motor supported in the housing, and a cartridge movably supported in the housing at a location between proximal and distal end portions of the housing. The proximal end portion of the housing defines an inlet for receiving a surgical instrument, and the distal end portion is configured to couple to a trocar. The housing defines a longitudinal channel therethrough. The cartridge stores a plurality of surgical clips and is configured to move, in response to an activation of the motor, relative to the housing between a first position and a second position. In the first position, a first surgical clip of the plurality of surgical clips is outside of the longitudinal channel of the housing. In the second position, the first surgical clip is within the longitudinal channel of the housing.

In aspects, the cartridge may be annular and may include a plurality of circumferentially spaced flanges that detachably support the respective plurality of surgical clips.

In aspects, the cartridge may define at least one slot configured to be coaxial with the longitudinal channel of the housing when the cartridge is in the first position.

In aspects, the reloading module may further include a sensor in communication with the motor. The motor may be configured to rotate the cartridge from the first position to the second position when the sensor determines that a distal end portion of the surgical instrument is moved proximally of the cartridge.

In aspects, the housing may include an intermediate portion positioned between the proximal and distal end portions. The intermediate portion may have an appendage extending laterally outward relative to the proximal and distal end portions. The cartridge may be housed within the appendage.

In aspects, the cartridge may be a rectangular strip that defines a linear array of openings configured for passage of the surgical instrument. Each of the openings may be positioned between adjacent surgical clips.

Further details and aspects of exemplary aspects of the disclosure are described in more detail below with reference to the appended figures.

As used herein, the terms parallel and perpendicular are understood to include relative configurations that are substantially parallel and substantially perpendicular up to about + or - 10 degrees from true parallel and true perpendicular.

### BRIEF DESCRIPTION OF THE DRAWINGS

Embodiments of the disclosure are described herein with reference to the accompanying drawings, wherein:
FIG. 1 is a schematic illustration of a surgical robotic system including a control tower, a console, and one or more surgical robotic arms;
FIG. 2 is a perspective view of a surgical robotic arm of the surgical robotic system of FIG. 1;
FIG. 3 is a perspective view of a setup arm with the surgical robotic arm of the surgical robotic system of FIG. 1;
FIG. 4 is a schematic diagram of a computer architecture of the surgical robotic system of FIG. 1;
FIG. 5 is a perspective view illustrating an aspect of a surgical implant reloading module coupled to a trocar of the surgical robotic system of FIG. 1;
FIG. 6 is a top view illustrating the reloading module of FIG. 5;
FIG. 7 is perspective view, with parts removed, illustrating internal components of the reloading module of FIG. 5;
FIG. 8 is a top, perspective view illustrating an exemplary carousel of the reloading module of FIG. 5;
FIG. 9A is a longitudinal, cross-sectional view of the reloading module of FIG. 5 illustrating a surgical instrument inserted in the reloading module;
FIG. 9B is a longitudinal, cross-sectional view of the reloading module of FIG. 5 illustrating the surgical instrument grasping a surgical implant from the reloading module;
FIG. 9C is a longitudinal, cross-sectional view of the reloading module of FIG. 5 illustrating the surgical instrument loaded with a surgical implant;
FIG. 9D is a front view illustrating the surgical instrument forming the surgical implant while positioned within the trocar of the surgical robotic system;
FIG. 10 is a flow chart illustrating an exemplary method of using the surgical implant reloading module of FIG. 5;
FIG. 11 is a top, perspective view illustrating another surgical implant reloading mechanism; and
FIG. 12 is a top, perspective view, with an outer housing shown in phantom, of the surgical implant reloading mechanism of FIG. 11.

### DETAILED DESCRIPTION

Embodiments of the disclosed surgical robotic system are described in detail with reference to the drawings, in which like reference numerals designate identical or corresponding elements in each of the several views. As used herein the term "distal" refers to that portion of the surgical robotic system or component thereof, that is closer to a patient, while the term "proximal" refers to that portion of the surgical robotic system or component thereof, that is further from the patient.

This disclosure describes a reloading module for detachable coupling to a surgical trocar or "port" of a surgical robotic system. The reloading module has stored therein a plurality of surgical implants, such as, for example, surgical clips for selective loading onto a surgical instrument, such as, for example, a single-fire surgical clip applier, during a robotically-guided surgical procedure. The reloading module allows for a reloading of the surgical instrument with a surgical implant without requiring the complete removal of the surgical instrument from a surgical field or detachment of the surgical instrument from the robotic arm. The reloading module may include a cartridge or carousel that holds the plurality of surgical implants and which moves or rotates the selected surgical implant into alignment with an axial path of the surgical instrument to allow for reloading of the surgical instrument.

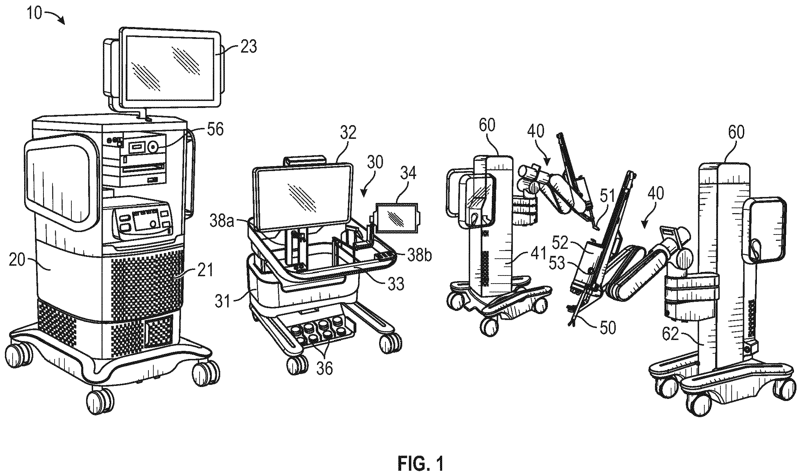

With reference to FIG. 1, a surgical robotic system 10 includes a control tower 20, which is connected to all of the components of the surgical robotic system 10 including a surgical console 30 and one or more robotic arms 40. Each of the robotic arms 40 includes a surgical instrument 50 removably coupled thereto. Each of the robotic arms 40 is also coupled to a movable cart 60.

The surgical instrument 50 is configured for use during minimally invasive surgical procedures. In embodiments, the surgical instrument 50 may be configured for open surgical procedures. In embodiments, the surgical instrument 50 may be an endoscope, such as an endoscopic camera 51, configured to provide a video feed for the user. In further embodiments, the surgical instrument 50 may be a surgical clip applier, a surgical stapler, an electrosurgical forceps, or the like.

One of the robotic arms 40 may include the endoscopic camera 51 configured to capture video of the surgical site. The endoscopic camera 51 may be a stereoscopic endoscope configured to capture two side-by-side (i.e., left and right) images of the surgical site to produce a video stream of the surgical scene. The endoscopic camera 51 is coupled to a video processing device 56, which may be disposed within the control tower 20. The video processing device 56 may be any computing device as described below configured to receive the video feed from the endoscopic camera 51 perform the image processing based on the depth estimating algorithms of the disclosure and output the processed video stream.

The surgical console 30 includes a first display 32, which displays a video feed of the surgical site provided by camera 51 of the surgical instrument 50 disposed on the robotic arms 40, and a second display 34, which displays a user interface for controlling the surgical robotic system 10. The first and second displays 32 and 34 are touchscreens allowing for displaying various graphical user inputs.

The surgical console 30 also includes a plurality of user interface devices, such as foot pedals 36 and a pair of handle controllers 38a and 38b which are used by a user to remotely control robotic arms 40. The surgical console further includes an armrest 33 used to support clinician's arms while operating the handle controllers 38a and 38b.

The control tower 20 includes a display 23, which may be a touchscreen, and outputs on the graphical user interfaces (GUIs). The control tower 20 also acts as an interface between the surgical console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgical console 30, in such a way that robotic arms 40 and the surgical instrument 50 execute a desired movement sequence in response to input from the foot pedals 36 and the handle controllers 38a and 38b.

Each of the control tower 20, the surgical console 30, and the robotic arm 40 includes a respective computer 21, 31, 41. The computers 21, 31, 41 are interconnected to each other using any suitable communication network based on wired or wireless communication protocols. The term "network," whether plural or singular, as used herein, denotes a data network, including, but not limited to, the Internet, Intranet, a wide area network, or a local area networks, and without limitation as to the full scope of the definition of communication networks as encompassed by the present disclosure. Suitable protocols include, but are not limited to, transmission control protocol/internet protocol (TCP/IP), datagram protocol/internet protocol (UDP/IP), and/or datagram congestion control protocol (DCCP). Wireless communication may be achieved via one or more wireless configurations, e.g., radio frequency, optical, Wi-Fi, Bluetooth (an open wireless protocol for exchanging data over short distances, using short length radio waves, from fixed and mobile devices, creating personal area networks (PANs), ZigBee^{®} (a specification for a suite of high level communication protocols using small, low-power digital radios based on the IEEE 122.15.4-2003 standard for wireless personal area networks (WPANs)).

The computers 21, 31, 41 may include any suitable processor (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to execute algorithms, calculations, and/or set of instructions described herein.

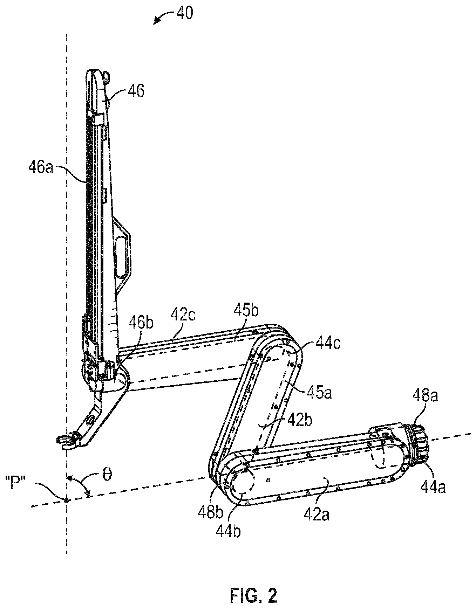

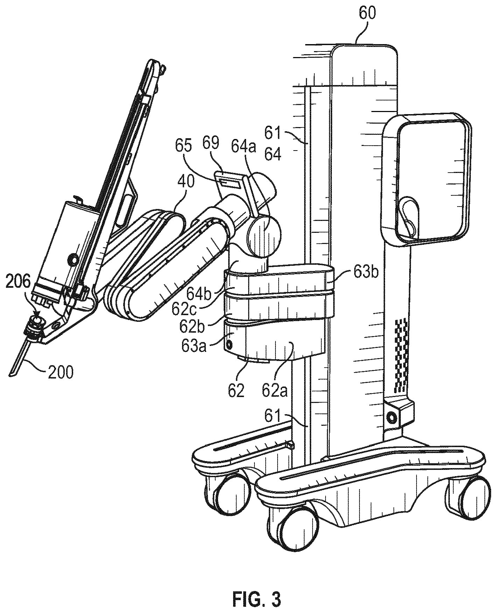

With reference to FIG. 2, each of the robotic arms 40 may include a plurality of links 42a, 42b, 42c, which are interconnected at joints 44a, 44b, 44c, respectively. The joint 44a is configured to secure the robotic arm 40 to the movable cart 60 and defines a first longitudinal axis. With reference to FIG. 3, the movable cart 60 includes a lift 61 and a setup arm 62, which provides a base for mounting of the robotic arm 40. The lift 61 allows for vertical movement of the setup arm 62. The movable cart 60 also includes a display 69 for displaying information pertaining to the robotic arm 40.

The setup arm 62 includes a first link 62a, a second link 62b, and a third link 62c, which provide for lateral maneuverability of the robotic arm 40. The links 62a, 62b, 62c are interconnected at joints 63a and 63b, each of which may include an actuator (not shown) for rotating the links 62b and 62b relative to each other and the link 62c. In particular, the links 62a, 62b, 62c are movable in their corresponding lateral planes that are parallel to each other, thereby allowing for extension of the robotic arm 40 relative to the patient (e.g., surgical table). In embodiments, the robotic arm 40 may be coupled to the surgical table (not shown). The setup arm 62 includes controls 65 for adjusting movement of the links 62a, 62b, 62c as well as the lift 61.

The third link 62c includes a rotatable base 64 having two degrees of freedom. In particular, the rotatable base 64 includes a first actuator 64a and a second actuator 64b. The first actuator 64a is rotatable about a first stationary arm axis which is perpendicular to a plane defined by the third link 62c and the second actuator 64b is rotatable about a second stationary arm axis which is transverse to the first stationary arm axis. The first and second actuators 64a and 64b allow for full three-dimensional orientation of the robotic arm 40.

The actuator 48b of the joint 44b is coupled to the joint 44c via the belt 45a, and the joint 44c is in turn coupled to the joint 46c via the belt 45b. Joint 44c may include a transfer case coupling the belts 45a and 45b, such that the actuator 48b is configured to rotate each of the links 42b, 42c and the holder 46 relative to each other. More specifically, links 42b, 42c, and the holder 46 are passively coupled to the actuator 48b which enforces rotation about a pivot point "P" which lies at an intersection of the first axis defined by the link 42a and the second axis defined by the holder 46. Thus, the actuator 48b controls the angle θ between the first and second axes allowing for orientation of the surgical instrument 50. Due to the interlinking of the links 42a, 42b, 42c, and the holder 46 via the belts 45a and 45b, the angles between the links 42a, 42b, 42c, and the holder 46 are also adjusted in order to achieve the desired angle θ. In embodiments, some or all of the joints 44a, 44b, 44c may include an actuator to obviate the need for mechanical linkages.

The joints 44a and 44b include an actuator 48a and 48b configured to drive the joints 44a, 44b, 44c relative to each other through a series of belts 45a and 45b or other mechanical linkages such as a drive rod, a cable, or a lever and the like. In particular, the actuator 48a is configured to rotate the robotic arm 40 about a longitudinal axis defined by the link 42a.

With reference to FIG. 2, the robotic arm 40 also includes a holder 46 defining a second longitudinal axis and configured to receive an instrument drive unit (IDU) 52 (FIG. 1). The IDU 52 is configured to couple to an actuation mechanism of the surgical instrument 50 and the camera 51 and is configured to move (e.g., rotate) and actuate the instrument 50 and/or the camera 51. IDU 52 transfers actuation forces from its actuators to the surgical instrument 50 to actuate components (e.g., end effector) of the surgical instrument 50. The holder 46 includes a sliding mechanism 46a, which is configured to move the IDU 52 along the second longitudinal axis defined by the holder 46. The holder 46 also includes a joint 46b, which rotates the holder 46 relative to the link 42c. During endoscopic procedures, the instrument 50 may be inserted through an endoscopic port or surgical trocar 200 (FIG. 3) held by the holder 46.

The robotic arm 40 also includes a plurality of manual override buttons 53 disposed on the IDU 52 and the setup arm 62, which may be used in a manual mode. The user may press one or more of the buttons 53 to move the component associated with the button 53.

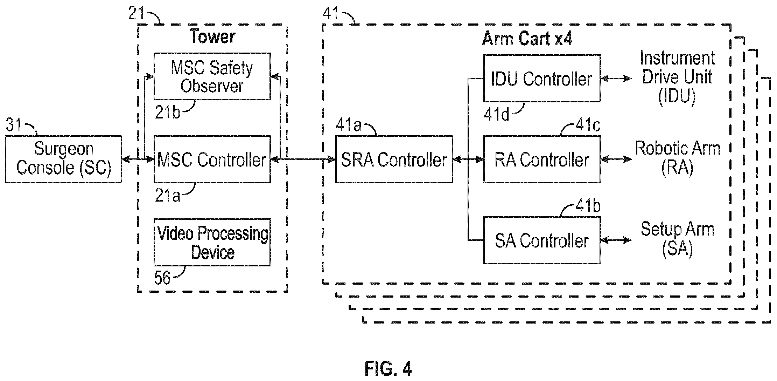

With reference to FIG. 4, each of the computers 21, 31, 41 of the surgical robotic system 10 may include a plurality of controllers, which may be embodied in hardware and/or software. The computer 21 of the control tower 20 includes a controller 21a and safety observer 21b. The controller 21a receives data from the computer 31 of the surgical console 30 about the current position and/or orientation of the handle controllers 38a and 38b and the state of the foot pedals 36 and other buttons. The controller 21a processes these input positions to determine desired drive commands for each joint of the robotic arm 40 and/or the IDU 52 and communicates these to the computer 41 of the robotic arm 40. The controller 21a also receives the actual joint angles measured by encoders of the actuators 48a and 48b and uses this information to determine force feedback commands that are transmitted back to the computer 31 of the surgical console 30 to provide haptic feedback through the handle controllers 38a and 38b. The safety observer 21b performs validity checks on the data going into and out of the controller 21a and notifies a system fault handler if errors in the data transmission are detected to place the computer 21 and/or the surgical robotic system 10 into a safe state.

The computer 41 includes a plurality of controllers, namely, a main cart controller 41a, a setup arm controller 41b, a robotic arm controller 41c, and an instrument drive unit (IDU) controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the movable cart 60, the robotic arm 40, and the IDU 52. The main cart controller 41a also communicates actual joint angles back to the controller 21a.

The setup arm controller 41b controls each of joints 63a and 63b, and the rotatable base 64 of the setup arm 62 and calculates desired motor movement commands (e.g., motor torque) for the pitch axis and controls the brakes. The robotic arm controller 41c controls each joint 44a and 44b of the robotic arm 40 and calculates desired motor torques required for gravity compensation, friction compensation, and closed loop position control of the robotic arm 40. The robotic arm controller 41c calculates a movement command based on the calculated torque. The calculated motor commands are then communicated to one or more of the actuators 48a and 48b in the robotic arm 40. The actual joint positions are then transmitted by the actuators 48a and 48b back to the robotic arm controller 41c.

The IDU controller 41d receives desired joint angles for the surgical instrument 50, such as wrist and jaw angles, and computes desired currents for the motors in the IDU 52. The IDU controller 41d calculates actual angles based on the motor positions and transmits the actual angles back to the main cart controller 41a.

The robotic arm 40 is controlled in response to a pose of the handle controller controlling the robotic arm 40, e.g., the handle controller 38a, which is transformed into a desired pose of the robotic arm 40 through a hand eye transform function executed by the controller 21a. The hand eye function, as well as other functions described herein, is/are embodied in software executable by the controller 21a or any other suitable controller described herein. The pose of one of the handle controller 38a may be embodied as a coordinate position and role-pitch-yaw ("RPY") orientation relative to a coordinate reference frame, which is fixed to the surgical console 30. The desired pose of the instrument 50 is relative to a fixed frame on the robotic arm 40. The pose of the handle controller 38a is then scaled by a scaling function executed by the controller 21a. In embodiments, the coordinate position is scaled down and the orientation is scaled up by the scaling function. In addition, the controller 21a also executes a clutching function, which disengages the handle controller 38a from the robotic arm 40. In particular, the controller 21a stops transmitting movement commands from the handle controller 38a to the robotic arm 40 if certain movement limits or other thresholds are exceeded and in essence acts like a virtual clutch mechanism, e.g., limits mechanical input from effecting mechanical output.

The desired pose of the robotic arm 40 is based on the pose of the handle controller 38a and is then passed by an inverse kinematics function executed by the controller 21a. The inverse kinematics function calculates angles for the joints 44a, 44b, 44c of the robotic arm 40 that achieve the scaled and adjusted pose input by the handle controller 38a. The calculated angles are then passed to the robotic arm controller 41c, which includes a joint axis controller having a proportional-derivative (PD) controller, the friction estimator module, the gravity compensator module, and a two-sided saturation block, which is configured to limit the commanded torque of the motors of the joints 44a, 44b, 44c.

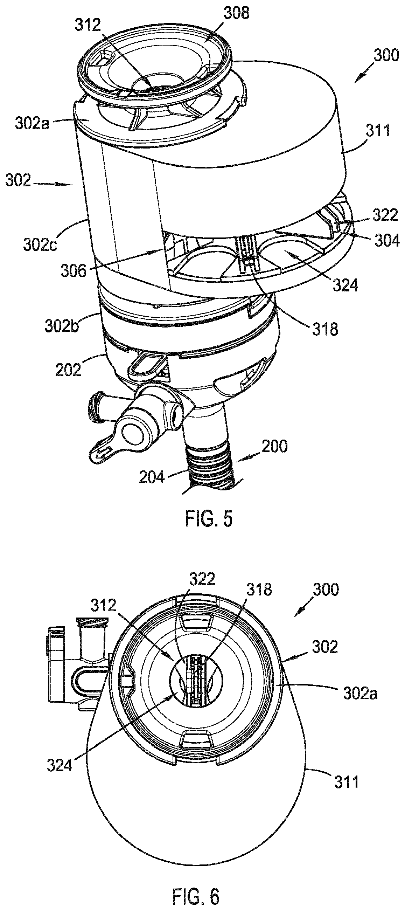

With reference to FIGS. 3 and 5, the surgical trocar 200 of the surgical robotic system 10 is configured for guiding the surgical instrument 50 through a natural or artificial (e.g., percutaneous) opening in a patient and into a surgical site or internal body cavity of the patient. The trocar 200 generally includes a head 202 and a cannula 204 extending distally from the head 202. The head 202 and cannula 204 collectively define a channel 206 (FIG. 3) longitudinally therethrough configured to receive the surgical instrument 50.

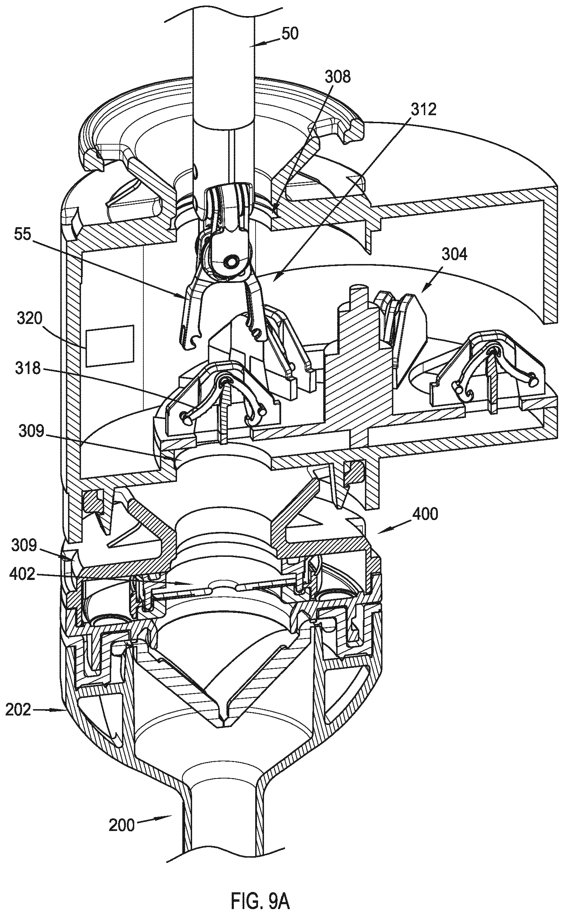

With reference to FIGS. 5-9A, the surgical robotic system 10 (FIG. 1) further includes a surgical implant reloading module 300 configured to be detachably coupled to the head 202 of the trocar 200. For example, the reloading module 300 may be threadedly coupled to the head 202, snap-fittingly engaged to the head 202, coupled to the head via latches, or coupled to the head 202 via any suitable fastening engagement. The reloading module 300 generally includes a main body or housing 302 and a cartridge 304, such as, for example, a carousel or wheel, movably supported within a cavity 306 of the housing 302. The housing 302 includes a proximal end portion 302a, a distal end portion 302b, and an intermediate portion 302c positioned between the proximal and distal end portions 302a, 302b. The proximal end portion 302a defines an inlet 308, and the distal end portion 302b defines an outlet 309 (FIG. 9A) configured to be in fluid communication with an inlet of the head 202 of the trocar 200. In aspects, a sterile interface or adapter assembly 400 (FIG. 9A) may be provided between the outlet 309 of the reloading module 300 and the head 202 of the trocar 200 to provide a fluid-sealed pathway from the reloading module 200 to the trocar 200. In aspects, the sterile adapter assembly 400 may have a seal 402 (FIG. 9A) positioned therein.

The intermediate portion 302c of the housing 302 has an appendage 311 extending laterally outward relative to the proximal and distal end portions 302a, 302b. The intermediate portion 302c defines the cavity 306 of the housing in which the cartridge 304 is housed. The housing 302 defines a longitudinally-extending channel 312 therethrough configured to be coaxial with the cannula 204 of the trocar 200 when the reloading module 300 is coupled to the head 202 of the trocar 200. The channel 312 is configured for passage of the surgical instrument 50 (FIG. 1), e.g., a surgical clip applier. In aspects, the housing 302 may define a longitudinally-extending lateral slot (not explicitly shown) in communication with the channel 312 to allow for lateral insertion and withdrawal of the surgical instrument 50 from the reloading module 300.

The cartridge 304 of the reloading module 300 is rotatably supported in the cavity 306 of the housing 302. In aspects, the cartridge 304 may be suspended within the cavity 306 by a spindle and/or supported on a bearing within the housing 302. In aspects, the cartridge 304 may be slidably or pivotably coupled to the housing 302. The cartridge 304 is configured to store a plurality of surgical implants 318, such as, for example, surgical clips. Other types of surgical implants 318 may be stored by the cartridge 304, such as, for example, staples, sutures, suture needles, or the like. The cartridge 304 may be annular and includes a plurality of circumferentially spaced flanges 322 extending radially outward from the center or hub thereof. Each flange 322 may define a pocket 326 therein having a respective surgical implant 318 removably secured therein.

The cartridge 304 defines one or more slots or openings 324 therein having a substantially similar diameter as a diameter of the channel 312 of the housing 302. Each slot 324 is positioned between adjacent flanges 322 of the cartridge 304 such that the flanges 322 and slots 324 alternate in succession in a circumferential direction of the cartridge 304. As shown, the slots 324 may be opened at an outer periphery of the cartridge 304 to allow for lateral removal of the cartridge 304 while a surgical instrument is received through one of the slots 324. In other aspects, the slots 324 may be closed.

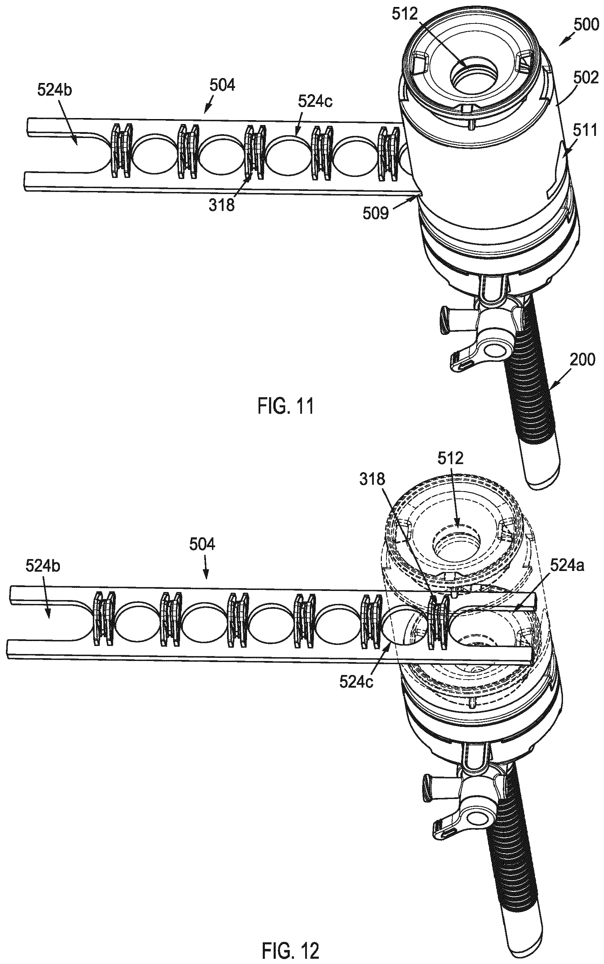

In aspects, the cartridge 304 may assume any suitable shape, such as, for example, a rectangular strip (FIGS. 11 and 12), star-shaped, triangular-shaped, or the like.

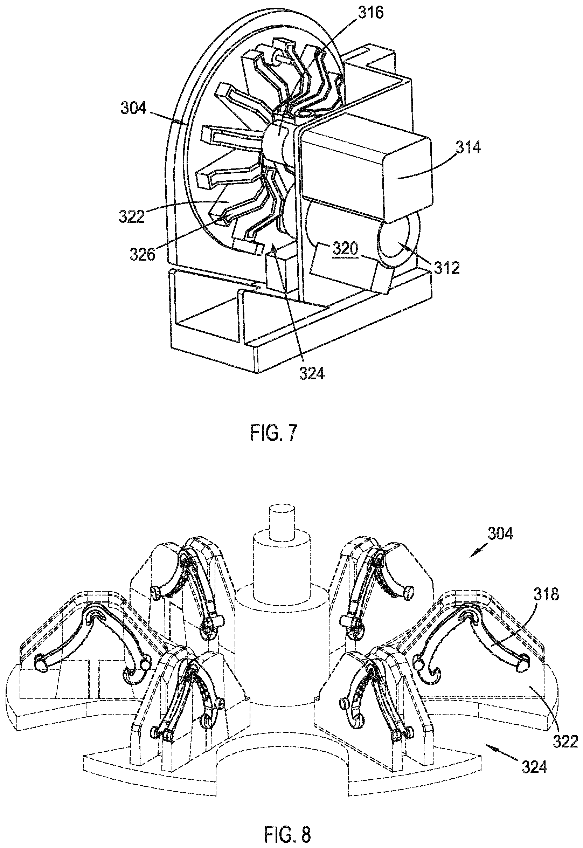

The reloading module 300 may include a motor 314 (FIG. 7) operably coupled to the cartridge 304 and configured to rotate the cartridge 304 about a central axis defined by the cartridge 304. The motor 314 may be powered by any suitable power source, such as, for example, a battery within the reloading module 300, electrical contacts in a trocar port latch, an external power source, or the like. The motor 314 may have a motor shaft 316 extending through a central point of the cartridge 304 and may be non-rotatably fixed thereto. In aspects, the cartridge 304 may be indirectly operably coupled to the motor shaft 316, such as, for example, the cartridge 304 may include a gear (not explicitly shown) and the motor shaft 316 may have a gear (not explicitly shown) in meshing engagement with the gear of the cartridge 304 for rotating the cartridge 304. Other operably engagements between the cartridge 304 and the motor 314 are contemplated, such as, for example, a belt drive.

The cartridge 304 is configured to move (e.g., rotate), in response to an activation of the motor 314 or via a manual actuation, between a first position and at least one second position. In the first position, one of the slots 324 of the cartridge 304 is coaxial with the longitudinal channel 312 of the housing 302 allowing for the free translation of the surgical instrument 50 therethrough. In the second position, as illustrated in FIG. 6, one of the surgical implants 318 is within the longitudinal channel 312 of the housing 302 corresponding with a loading condition of the reloading module 300.

The reloading module 300 may further include a sensor/encoder 320 (FIG. 7) in wired or wireless communication with the motor 314 and/or at least one of the processors of the computers 21, 31, or 41 (FIG. 4) of the surgical robotic system 10. Wireless communication includes radio frequency, optical, WIFI, Bluetooth^{®} (an open wireless protocol for exchanging data over short distances (using short length radio waves) from fixed and mobile devices, ZigBee^{®} (a specification for a suite of high level communication protocols using small, low-power digital radios based on the IEEE 802.15.4-2003 standard for wireless personal area networks (WPANs)), etc. The sensor 320 may be any suitable sensor and/or encoder configured to sense movement or a position of a selected location(s) of the surgical instrument 50 (FIG. 2). Non-limiting examples of such include rotary and linear encoders, Hall effect and other magnetic-based sensors, linearly variable displacement transducers, optical sensors, etc.

The surgical instrument 50 (FIG. 2) may be equipped with a sensed element (e.g., a magnetic element, an encoder, a light, etc.) attached to a selected location of the surgical instrument 50. For example, the sensed element (not explicitly shown) may be attached to the end effector 55 (FIG. 9A) of the surgical instrument 50 and the sensor 320 of the reloading module 300 may be positioned within the proximal end portion 302a of the housing 302 at a location proximal of the cartridge 304. As such, upon the end effector of the surgical instrument 50 moving proximally through the reloading module 300 to a position proximal of the cartridge 304, the sensor 320 causes the motor 314 to rotate the cartridge 304 from the first position to the second position to allow for reloading the end effector with the surgical implant 318.

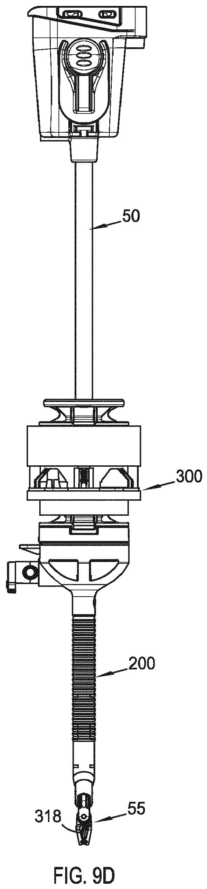

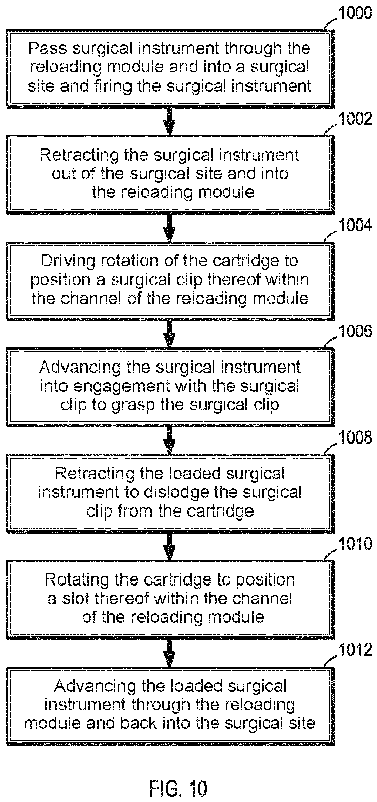

In operation, the reloading module 300, pre-filled with a plurality of surgical implants 318 (e.g., surgical clips), may be secured to the head 202 of the trocar 200, as shown in FIG. 9A. With the cartridge 304 of the reloading module 300 in the first position, in which one of the slots 324 is coaxial with the longitudinal channel 312 of the reloading module 300, in step 1000 (FIG. 10), the surgical instrument 50 may be passed through the longitudinal channel 312 of the reloading module 300, through the trocar 200, and into a surgical site, as shown in FIG. 9D. After firing a surgical implant 318 from the surgical instrument 50 into tissue, in step 1002, the now spent surgical instrument 50 may be translated proximally out of the surgical site, through the trocar 200, and stop when the end effector 55 of the surgical instrument 50 is within the reloading module 300 and proximal of the cartridge 304, as shown in FIG. 9A.

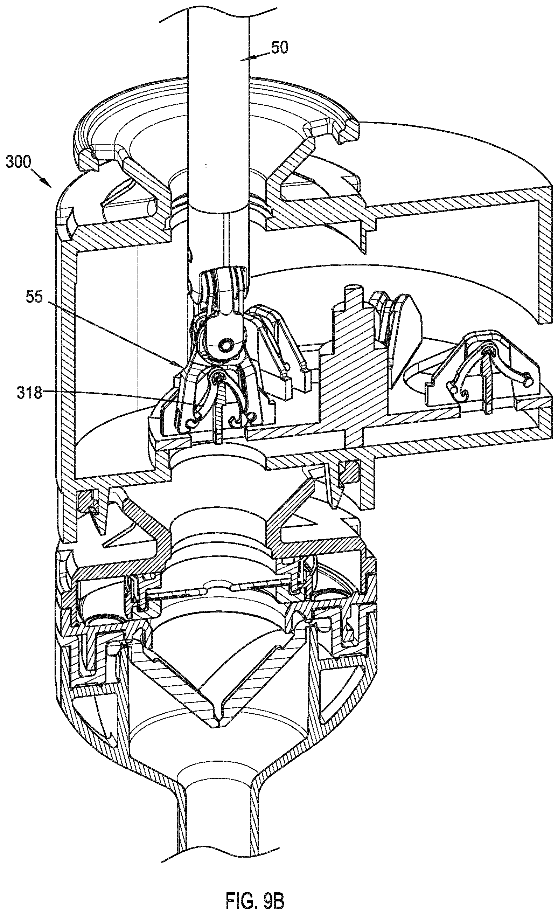

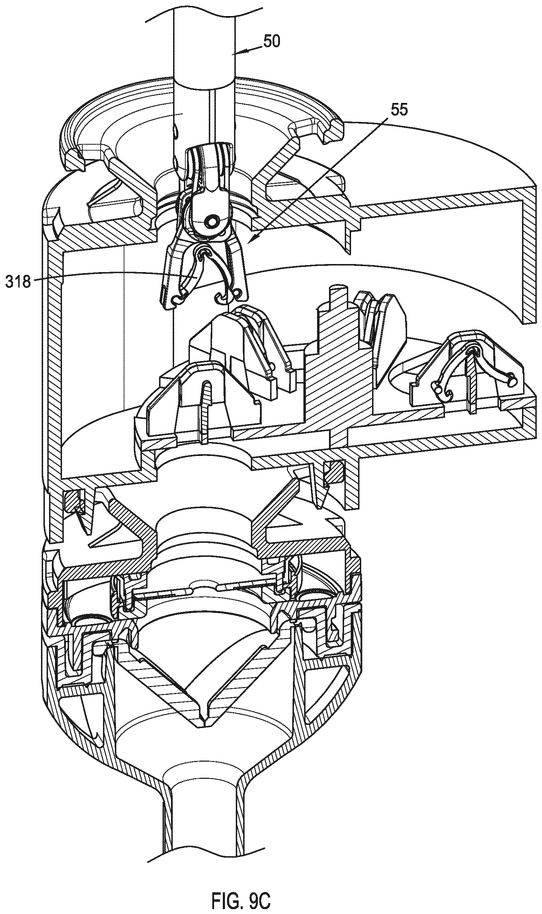

Upon the end effector 55 of the surgical instrument 50 moving to a location adjacent the sensor 320, and therefore proximally of the cartridge 304 while remaining within the reloading module 300, in step 1004, the sensor 320 communicates to the motor 314 to drive a rotation of the cartridge 304 from the first position to the second position, in which one of the surgical implants 318 of the reloading module 300 is within the longitudinal channel 312 of the reloading module 300. With the cartridge 304 in the second position, and jaws of the end effector 55 of the surgical instrument 50 in a rotational orientation matching a rotational orientation of the surgical implant 318 (e.g., a surgical clip), in step 1006, the surgical instrument 50 is translated distally to engage the jaws of the end effector 55 with the surgical implant 318, as shown in FIG. 9B. With the surgical implant 318 captured between the jaws of the end effector of the surgical instrument 50, in step 1008, the surgical instrument 50 is retracted to dislodge the surgical implant 318 from the cartridge 304, as shown in FIG. 9C. The surgical instrument 50, with the surgical implant secured thereto, is retracted a sufficient distance from the cartridge 304, without exiting the reloading module 300. In other aspects, the surgical instrument 50 may be retracted out of the reloading module 300.

In step 1010, the cartridge 304 is then rotated to a position in which one of the slots 324 of the cartridge 304 is coaxial with the longitudinal channel 312 of the housing 302. With the slot 324 coaxial with the longitudinal channel 312 of the housing 302, in step 1012, the surgical instrument 50 is moved distally through the reloading module 300, the trocar 200, and back into the surgical site for continued tissue treatment, as shown in FIG. 9D.

By allowing for extracorporeal reloading of the surgical instrument 50, the surgical instrument 50 may be shorter than ordinary given that the surgical instrument 50 does not need to be equipped with a cartridge loaded with surgical implants 318. Additionally, due to the reloading module 300, reloading a single-fire surgical instrument (e.g., a surgical clip applier) does not require the complete removal of the surgical instrument from the sterile field, or the complete removal of the surgical instrument from within the trocar 200 or reloading module 300, thereby expediting the surgical procedure. The reloading module 300 also makes removal of the surgical instrument from the robotic arm unnecessary, which would otherwise result in the need for a recalibration.

With reference to FIGS. 11 and 12, another type of surgical implant reloading mechanism 500 of the surgical robotic system 10 of FIG. 1 is shown. The surgical implant reloading mechanism 500 is similar to and may include any of the features of the surgical implant reloading mechanism 300 of FIG. 5 except as explicitly contradicted below.

The surgical implant reloading mechanism 500 includes a housing 502 and a cartridge, such as, for example, a rectangular-shaped strip 504 that extends perpendicularly through the housing 502. The housing 502 defines a longitudinally-extending channel 512 configured for passage of a surgical instrument, such as, for example, surgical instrument 50 (FIG. 1). The housing 502 may be detachably connected to or monolithically formed with the trocar 200. The strip 504 may be plate-like and/or assume other suitable shapes besides rectangular.

The strip 504 detachably supports a plurality of surgical implants, such as, for example, surgical clips 318. The strip 504 defines a linear array of spaced-apart openings 524a, 524b, 524c configured for passage of the surgical instrument 50. Each of the openings 524c is positioned between adjacent surgical clips 318. It is contemplated that first and second end-most openings 524a, 524b of the strip 504 may be opened whereas inner openings 524c may be closed.

The housing 502 may define a pair of diametrically opposed openings 509, 511 in an outer surface thereof to allow for the strip 504 to pass through either side of the housing 502 during use. The strip 504 may be axially supported within the housing 502 by a track (not explicitly shown), wheels, bearings, or the like. As such, the strip 504 may be translated in a direction perpendicular to a longitudinal axis defined by the channel 512 between a first position and a second position. In the first position (FIG. 12), one of the openings of the strip 504 (e.g., the end-most opening 524a) is coaxial with the longitudinal channel 512 of the reloading module 500 to allow for passage of the surgical instrument 50 into and out of the surgical site.

In the second position (not explicitly shown), one of the surgical clips 318 of the strip 504 is positioned within the longitudinal channel 512 to allow for loading the surgical instrument 50 with the surgical clip 318. Upon loading the surgical instrument 50 with the surgical clip 318, the strip 304 may be further translated to position another opening (e.g., the inner opening 524c positioned adjacent the end-most opening 524a) in coaxial alignment with the longitudinal channel 512. This sequence may be continued until each of the surgical implants 318 of the strip 504 have been used and the second end-most opening 524b is coaxial with the longitudinal channel 512.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

The invention may be described by reference to the following numbered paragraphs:-
1. A surgical robotic system, comprising:
   a surgical robotic arm having an elongated rail configured to movably support a surgical instrument;
   a trocar including:
      a head configured for attachment to the elongated rail; and
      a cannula extending distally from the head and configured to receive the surgical instrument; and
   a reloading module configured to couple to the head of the trocar and including:
      a housing defining a longitudinal channel configured to be coaxial with the cannula of the trocar when the reloading module is coupled to the head of the trocar; and
      a cartridge movably coupled to the housing and configured to store a plurality of surgical implants, wherein the cartridge is configured to move relative to the housing to position a surgical implant of the plurality of surgical implants within the longitudinal channel of the housing.
2. The surgical robotic system according to paragraph 1, wherein the cartridge is axially or rotatably supported in the housing such that the cartridge slides or rotates relative to the housing from a first position, in which the surgical implant is positioned outside of the longitudinal channel of the housing, to a second position, in which the surgical implant is within the longitudinal channel of the housing.
3. The surgical robotic system according to paragraph 2, wherein the cartridge is annular and includes a plurality of circumferentially spaced flanges that detachably support the respective plurality of surgical implants.
4. The surgical robotic system according to paragraph 3, wherein the cartridge defines at least one slot configured to be coaxial with the longitudinal channel of the housing when the cartridge is in the first position.
5. The surgical robotic system according to paragraph 4, wherein the at least one slot is a plurality of slots, each slot of the plurality of slots being positioned between adjacent flanges of the plurality of flanges.
6. The surgical robotic system according to paragraph 1, wherein the reloading module further includes a motor operably coupled to the cartridge and configured to move the cartridge to change the position of the surgical implant relative to the longitudinal channel of the housing.
7. The surgical robotic system according to paragraph 6, wherein the reloading module further includes a sensor in communication with the motor, the motor being configured to move the cartridge to position the surgical implant within the longitudinal channel when the sensor determines that a distal end portion of the surgical instrument is moved proximally of the cartridge.
8. The surgical robotic system according to paragraph 1, wherein the plurality of surgical implants includes at least one of staples, clips, or sutures.
9. A surgical robotic system, comprising:
   a trocar including:
      a head configured for attachment to a surgical robotic arm; and
      a cannula extending distally from the head and configured to receive a surgical instrument; and
   a reloading module configured to couple to the head of the trocar and including:
      a housing defining a longitudinal channel configured to be coaxial with the cannula of the trocar when the reloading module is coupled to the head of the trocar; and
      a cartridge movably coupled to the housing and configured to store a plurality of surgical implants, wherein the cartridge is configured to move relative to the housing between a first position, in which a surgical implant of the plurality of surgical implants is outside of the longitudinal channel of the housing, and a second position, in which the surgical implant is within the longitudinal channel of the housing.
10. The surgical robotic system according to paragraph 9, wherein the cartridge is annular and includes a plurality of circumferentially spaced flanges that detachably support the respective plurality of surgical implants.
11. The surgical robotic system according to paragraph 10, wherein the cartridge defines at least one slot configured to be coaxial with the longitudinal channel of the housing when the cartridge is in the first position.
12. The surgical robotic system according to paragraph 11, wherein the at least one slot is a plurality of slots, each slot of the plurality of slots being positioned between adjacent flanges of the plurality of flanges.
13. The surgical robotic system according to paragraph 9, wherein the reloading module further includes a motor operably coupled to the cartridge and configured to rotate the cartridge between the first and second positions.
14. The surgical robotic system according to paragraph 13, wherein the reloading module further includes a sensor in communication with the motor, the motor being configured to rotate the cartridge from the first position to the second position when the sensor determines that a distal end portion of the surgical instrument is moved proximally of the cartridge.
15. A reloading module for use in a surgical robotic system, the reloading module comprising:
   a housing having a proximal end portion defining an inlet for receiving a surgical instrument, and a distal end portion configured to couple to a trocar, the housing defining a longitudinal channel therethrough;
   a motor supported in the housing; and
   a cartridge movably supported in the housing at a location between the proximal and distal end portions, the cartridge storing a plurality of surgical clips, wherein the cartridge is configured to move, in response to an activation of the motor, relative to the housing between a first position, in which a first surgical clip of the plurality of surgical clips is outside of the longitudinal channel of the housing, and a second position, in which the first surgical clip is within the longitudinal channel of the housing.
16. The reloading module according to paragraph 15, wherein the cartridge is annular and includes a plurality of circumferentially spaced flanges that detachably support the respective plurality of surgical clips.
17. The reloading module according to paragraph 16, wherein the cartridge defines at least one slot configured to be coaxial with the longitudinal channel of the housing when the cartridge is in the first position.
18. The reloading module according to paragraph 17, wherein the at least one slot is a plurality of slots, each slot of the plurality of slots being positioned between adjacent flanges of the plurality of flanges.
19. The reloading module according to paragraph 15, further comprising a sensor in communication with the motor, the motor being configured to rotate the cartridge from the first position to the second position when the sensor determines that a distal end portion of the surgical instrument is moved proximally of the cartridge.
20. The reloading module according to paragraph 15, wherein the cartridge is a rectangular strip that defines a linear array of openings configured for passage of the surgical instrument, each opening of the array of openings being positioned between adjacent surgical clips of the plurality of surgical clips.

## Claims

1. A surgical robotic system, comprising:
a surgical robotic arm having an elongated rail configured to movably support a surgical instrument;
a trocar including:
a head configured for attachment to the elongated rail; and
a cannula extending distally from the head and configured to receive the surgical instrument; and
a reloading module configured to couple to the head of the trocar and including:
a housing defining a longitudinal channel configured to be coaxial with the cannula of the trocar when the reloading module is coupled to the head of the trocar; and
a cartridge movably coupled to the housing and configured to store a plurality of surgical implants, wherein the cartridge is configured to move relative to the housing to position a surgical implant of the plurality of surgical implants within the longitudinal channel of the housing.

2. The surgical robotic system according to claim 1, wherein the cartridge is axially or rotatably supported in the housing such that the cartridge slides or rotates relative to the housing from a first position, in which the surgical implant is positioned outside of the longitudinal channel of the housing, to a second position, in which the surgical implant is within the longitudinal channel of the housing.

3. The surgical robotic system according to claim 2, wherein the cartridge is annular and includes a plurality of circumferentially spaced flanges that detachably support the respective plurality of surgical implants.

4. The surgical robotic system according to claim 3, wherein the cartridge defines at least one slot configured to be coaxial with the longitudinal channel of the housing when the cartridge is in the first position; preferably wherein the at least one slot is a plurality of slots, each slot of the plurality of slots being positioned between adjacent flanges of the plurality of flanges.

5. The surgical robotic system according to any preceding claim, wherein the reloading module further includes a motor operably coupled to the cartridge and configured to move the cartridge to change the position of the surgical implant relative to the longitudinal channel of the housing; preferably wherein the reloading module further includes a sensor in communication with the motor, the motor being configured to move the cartridge to position the surgical implant within the longitudinal channel when the sensor determines that a distal end portion of the surgical instrument is moved proximally of the cartridge.

6. The surgical robotic system according to any preceding claim, wherein the plurality of surgical implants includes at least one of staples, clips, or sutures.

7. A surgical robotic system, comprising:
a trocar including:
a head configured for attachment to a surgical robotic arm; and
a cannula extending distally from the head and configured to receive a surgical instrument; and
a reloading module configured to couple to the head of the trocar and including:
a housing defining a longitudinal channel configured to be coaxial with the cannula of the trocar when the reloading module is coupled to the head of the trocar; and
a cartridge movably coupled to the housing and configured to store a plurality of surgical implants, wherein the cartridge is configured to move relative to the housing between a first position, in which a surgical implant of the plurality of surgical implants is outside of the longitudinal channel of the housing, and a second position, in which the surgical implant is within the longitudinal channel of the housing.

8. The surgical robotic system according to claim 7, wherein the cartridge is annular and includes a plurality of circumferentially spaced flanges that detachably support the respective plurality of surgical implants; preferably wherein the cartridge defines at least one slot configured to be coaxial with the longitudinal channel of the housing when the cartridge is in the first position.

9. The surgical robotic system according to claim 8, wherein the at least one slot is a plurality of slots, each slot of the plurality of slots being positioned between adjacent flanges of the plurality of flanges.

10. The surgical robotic system according to any of claims 7 to 9, wherein the reloading module further includes a motor operably coupled to the cartridge and configured to rotate the cartridge between the first and second positions; preferably wherein the reloading module further includes a sensor in communication with the motor, the motor being configured to rotate the cartridge from the first position to the second position when the sensor determines that a distal end portion of the surgical instrument is moved proximally of the cartridge.

11. A reloading module for use in a surgical robotic system, the reloading module comprising:
a housing having a proximal end portion defining an inlet for receiving a surgical instrument, and a distal end portion configured to couple to a trocar, the housing defining a longitudinal channel therethrough;
a motor supported in the housing; and
a cartridge movably supported in the housing at a location between the proximal and distal end portions, the cartridge storing a plurality of surgical clips, wherein the cartridge is configured to move, in response to an activation of the motor, relative to the housing between a first position, in which a first surgical clip of the plurality of surgical clips is outside of the longitudinal channel of the housing, and a second position, in which the first surgical clip is within the longitudinal channel of the housing.

12. The reloading module according to claim 11, wherein the cartridge is annular and includes a plurality of circumferentially spaced flanges that detachably support the respective plurality of surgical clips; preferably wherein the cartridge defines at least one slot configured to be coaxial with the longitudinal channel of the housing when the cartridge is in the first position.

13. The reloading module according to claim 12, wherein the at least one slot is a plurality of slots, each slot of the plurality of slots being positioned between adjacent flanges of the plurality of flanges; preferably further comprising a sensor in communication with the motor, the motor being configured to rotate the cartridge from the first position to the second position when the sensor determines that a distal end portion of the surgical instrument is moved proximally of the cartridge.

14. The reloading module according to any of claims 11 to 13, wherein the cartridge is a rectangular strip that defines a linear array of openings configured for passage of the surgical instrument, each opening of the array of openings being positioned between adjacent surgical clips of the plurality of surgical clips.
